# EUROPÄISCHE PATENTANMELDUNG

(11) **EP 4 275 714 A1**
(43) Veröffentlichungstag der Anmeldung: **15.11.2023**
(21) Anmeldenummer: 22173185.4
(22) Anmeldetag: 13.05.2022
(51) Int. Cl.: A61M 5/142, A61M 5/145, A61M 5/148, A61M 5/168

(54) **VORRICHTUNG UND VERFAHREN ZUR DOSIERTEN ABGABE EINER FLÜSSIGKEIT**

(71) Anmelder: Heraeus Medical GmbH, 61273 Wehrheim (DE)
(72) Erfinder: Vogt, Sebastian, 61273 Wehrheim (DE); Kluge, Thomas, 61273 Wehrheim (DE)
(74) Vertreter: Heraeus IP

(57) **Zusammenfassung**

Die Erfindung betrifft eine Vorrichtung und ein Verfahren zur dosierten Abgabe einer Flüssigkeit zwecks Wirkstofffreisetzung im menschlichen oder tierischen Körper sowie eine Verwendung einer Vorrichtung.

Eine Vorrichtung (10) zur dosierten Abgabe einer Flüssigkeit (12) zwecks Wirkstofffreisetzung im menschlichen oder tierischen Körper umfasst eine Dosierungseinrichtung (14) zur Dosierung eines definierten Volumens einer Flüssigkeit (12), einen mit der Dosierungseinrichtung (14) in Fluidverbindung stehenden Einlass (16) zum Zuführen von Flüssigkeit (12) in die Dosierungseinrichtung (14) sowie einen mit der Dosierungseinrichtung (14) in Fluidverbindung stehenden Auslass (18) zur Abgabe des definierten Volumens der Flüssigkeit (12). Die Dosierungseinrichtung (14) umfasst eine Wandung (21), die einen Dosierungshohlraum (20) zur Aufnahme der Flüssigkeit (12) umschließt. Durch manuellen Druck auf die Wandung (21) kann der Dosierungshohlraum (20) verkleinert werden, sodass das definierte Volumen der Flüssigkeit (12) aus dem Dosierungshohlraum (20) zum Auslass (18) gefördert wird.

## Beschreibung

Die Erfindung betrifft eine Vorrichtung und ein Verfahren zur dosierten Abgabe einer Flüssigkeit zwecks Wirkstofffreisetzung im menschlichen oder tierischen Körper sowie eine Verwendung einer Vorrichtung.

Rheumatische Erkrankungen gehören zur den sogenannten Autoimmunerkrankungen, bei denen das Immunsystem aus noch nicht genau bekannten Ursachen körpereigene Strukturen angreift. Die rheumatoide Arthritis und die Psoriasis-Arthritis gehören zum rheumatischen Formenkreis und sind mit Gelenkentzündungen oder Arthritiden verbunden, die meist chronisch verlaufen und zur fortschreitenden Zerstörung der Gelenke führen können. Für die systemische medikamentöse Therapie der rheumatoiden Arthritis und die Psoriasis-Arthritis stehen eine Reihe von Wirkstoffgruppen zur Verfügung, beispielsweise Analgetika, nicht-steroidale Antiphlogistika, Glucocorticoide und Basistherapeutika (Disease modifying anti-rheumatic drugs, DMARD) unterteilt. Daneben gibt es moderne Antikörper (Biologicals), welche das α-TNF (a-Tumornekrosefaktor) oder das Interleukin IL-17 blockieren. Mit der systemischen medikamentösen Therapie können jedoch nur relativ geringe Serumkonzentrationen der Wirkstoffe erreicht werden, da anderenfalls unerwünschte Nebenwirkungen auftreten, wie zum Beispiel Schädigungen der Nieren und der Leber. Höhere Serumkonzentrationen werden daher in der Praxis zumeist vermieden.

Das hat zur Folge, dass am Bestimmungsort, z. B. im Gewebe der Gelenkkapsel und an Sehnenansätzen, die Wirkstoffkonzentrationen nur sehr niedrig sein können und daher nur ein begrenzter immunmodulierender Effekt möglich ist. Zur Verminderung der chronischen Entzündungsprozesse im Gelenkbereich von Patienten mit rheumatoider Arthritis und mit Psoriasis-Arthritis sowie bei vielen anderen Krankheiten wäre es daher wünschenswert, wenn die entsprechenden Wirkstoffe lokal in der Nähe der Gelenkkapsel und/oder betroffener Sehnen bzw. Sehnenansätze und insbesondere direkt am betroffenen Körperstruktur appliziert werden könnten, um eine lokal hohe Konzentration dieser Wirkstoffe zu erreichen. Daneben ist eine Vielzahl weiterer Krankheitsbilder bekannt, bei denen mit systemischer Gabe von Wirkstoffen lediglich vergleichsweise geringe Konzentrationen am Zielort erreichbar sind, obwohl höhere lokale Konzentrationen wünschenswert wären.

Insbesondere bei rheumatischen Erkrankungen, zur Schmerztherapie, zur lokalen Zytostatika-Freisetzung und zur Behandlung von Tumorerkrankungen wäre es ferner wünschenswert, wenn die betroffenen Patienten selbst die Wirkstofflösungen bei Bedarf applizieren können.

Ein medizinisches Flüssigkeitsdepot, welches unter die Haut implantiert und mit einer Kanüle aufgefüllt werden kann, ist aus der Publikation EP 3 978 065 A1 bekannt.

Die Aufgabe der Erfindung besteht darin, eine Vorrichtung, ein Verfahren und eine Verwendung zur Verfügung zu stellen, mit denen eine dosierte Abgabe einer Flüssigkeit zwecks Wirkstofffreisetzung im menschlichen oder tierischen Körper möglich ist und die zumindest einen Teil der genannten Nachteile behebt. Insbesondere soll ein Patient selbst in die Lage versetzt werden, bei Bedarf Wirkstoff freizusetzen.

Die Aufgabe wird gelöst durch eine Vorrichtung gemäß Anspruch 1 sowie ein Verfahren und eine Verwendung gemäß den nebengeordneten Ansprüchen. Vorteilhafte Ausgestaltungen sind in den Unteransprüchen angegeben.

Zur Lösung der Aufgabe dient eine Vorrichtung zur dosierten Abgabe einer Flüssigkeit zwecks Wirkstofffreisetzung im menschlichen oder tierischen Körper. Die Vorrichtung umfasst eine Dosierungseinrichtung zur Dosierung eines definierten Volumens einer Flüssigkeit, einen mit der Dosierungseinrichtung in Fluidverbindung stehenden Einlass zum Zuführen von Flüssigkeit in die Dosierungseinrichtung sowie einen mit der Dosierungseinrichtung in Fluidverbindung stehenden Auslass zur Abgabe des definierten Volumens der Flüssigkeit. Die Dosierungseinrichtung umfasst eine Wandung, die einen Dosierungshohlraum zur Aufnahme der Flüssigkeit umschließt. Durch manuellen Druck auf die Wandung kann der Dosierungshohlraum verkleinert werden, sodass das definierte Volumen der Flüssigkeit aus dem Dosierungshohlraum zum Auslass gefördert wird.

Die Vorrichtung ist in der Lage, durch manuellen Druck und damit auf einfache und reproduzierbare Weise ein definiertes Volumen einer Flüssigkeit zu dosieren. Auf diese Weise ist eine einfache und sichere Wirkstoffabgabe möglich, insbesondere durch den Patienten selbst. Dabei kann der Patient den Zeitpunkt der Abgabe der Wirkstofflösung selbst bestimmen.

Der manuelle Druck kann mit einem Finger ausgeübt werden. Der manuelle Druck wird auf zumindest einen Bereich der Wandung ausgeübt. Durch den Einlass kann der Dosierungseinrichtung Wirkstofflösung zugeführt werden, sodass eine mehrfache bzw. wiederholte Wirkstoffabgabe möglich ist. Der Auslass kann in der Nähe des Bestimmungsortes der Wirkstofffreisetzung angeordnet werden, insbesondere im Körper des Patienten. Ebenso kann ein geeignetes Leitungselement zwischen dem Auslass und dem Bestimmungsort angeordnet werden. So kann lokal eine hohe Wirkstoffkonzentration erreicht werden, ohne dabei die bekannten Nachteile einer hohen Serumkonzentration in Kauf nehmen zu müssen. Darüber hinaus ist die Vorrichtung aufgrund ihrer einfachen mechanischen Funktionsweise einfach und robust, kostengünstig in der Herstellung sowie sicher in der Anwendung.

In einer Ausführungsform ist die Vorrichtung so eingerichtet, dass sie unter der Haut (subkutan) implantiert werden kann. Alternativ oder zusätzlich kann die Vorrichtung so eingerichtet sein, dass sie auf der Haut befestigt werden kann, beispielsweise durch Aufkleben.

Die Flüssigkeit enthält insbesondere zumindest einen Wirkstoff. Eine Flüssigkeit im Sinne der Erfindung umfasst jede Art der Lösung mit einem flüssigen Lösungsmittel.

Der Dosierungshohlraum ist ein Hohlraum, der zur Dosierung dient. Der Dosierungshohlraum wird durch manuellen Druck verkleinert. Damit ist gemeint, dass das innere Volumen des Dosierungshohlraum, in dem Flüssigkeit enthalten sein kann, verringert wird. Dadurch wird Flüssigkeit aus dem Dosierungshohlraum verdrängt und gelangt zum Auslass. Mit anderen Worten ist die Dosierungseinrichtung eine manuell bedienbare mechanische Pumpe. Der manuelle Druck erfolgt auf zumindest einen Bereich der Wandung. Insbesondere kann der Dosierungshohlraum im Anschluss wieder vergrößert und wieder mit der Flüssigkeit aufgefüllt werden, sodass eine mehrfache Dosierung möglich ist. Flüssigkeit kann also durch den Einlass in den Dosierungshohlraum fließen und von dort in dosierter Weise durch den Auslass abgegeben werden.

Der Dosierungshohlraum ist so bemessen, dass er bei manuellem Druck auf die Wandung das definierte Volumen der Flüssigkeit abgibt. Es ist nicht ausgeschlossen, dass dabei ein Restvolumen im Dosierungshohlraum verbleibt. Das nutzbare Volumen entspricht also nicht notwendigerweise dem absoluten Volumen des Dosierungshohlraums. Durch den manuellen Druck wird das definierte Volumen der Flüssigkeit insbesondere zum Auslass gedrückt. Insbesondere ist der Dosierungshohlraum scheibenförmig und/oder hat in Draufsicht eine kreisrunde Form. Insbesondere hat der Dosierungshohlraum eine plane Unterseite und/oder eine konvex gewölbte Oberseite. Die Form des Dosierungshohlraums kann demnach eine plankonvexe Form sein.

Das definierte Volumen der Flüssigkeit wird aus dem Auslass der Vorrichtung abgegeben. Die Abgabe kann direkt in den Körper des Patienten erfolgen. Die Vorrichtung kann auch ein oder mehrere Leitungselemente umfassen, in das die Flüssigkeit zunächst abgegeben wird und aus dem die Flüssigkeit dann in den Körper des Patienten abgegeben wird. Ein Leitungselement kann beispielswiese ein Schlauch sein. Die Abgabe dient insbesondere dem gezielten Einbringen der Flüssigkeit in einen Bereich eines Körpers eines Patienten, z. B. in die Nähe einer Gelenkkapsel, einer Sehne und/oder eines Sehnenansatzes.

Der Einlass und der Auslass ist eine Öffnung, die eine Fluidverbindung zum Dosierungshohlraum ermöglicht. Beim Einlass und/oder beim Auslass kann eine Anschlussmöglichkeit für ein Leitungselement vorhanden sein. Die Vorrichtung ist geeignet zur dosierten Abgabe einer Flüssigkeit zwecks Wirkstofffreisetzung im menschlichen oder tierischen Körper. Die Wirkstofffreisetzung muss dabei nicht notwendigerweise im Körper, sondern kann selbstverständlich auch am jeweiligen Körper erfolgen.

In einer Ausgestaltung ist zumindest ein Bereich der Wandung so ausgebildet, dass zumindest der Bereich der Wandung durch den manuellen Druck elastisch verformt wird. Mit anderen Worten ist die Wandung zumindest teilweise elastisch verformbar. Auf diese Weise ist die Verkleinerung des Dosierungshohlraums auf besonders einfache und zuverlässige Weise möglich. Insbesondere besteht die Wandung zumindest einer Seite des Dosierungshohlraums zumindest im Wesentlichen aus einem elastisch verformbaren Material. In einer Ausführungsform besteht die Wandung des gesamten Dosierungshohlraums teilweise oder vollständig aus einem elastisch verformbaren Material. So kann bei Änderung des Füllstands des Dosierungshohlraum auf einfache Weise eine Volumenänderung erfolgen. In einer Ausführungsform umfasst die Wandung des Dosierungshohlraums eine im Wesentlichen starre Bodenplatte und ein mit dieser mechanisch verbundenes elastisches Abdeckelement. Eine Bodenplatte und ein Abdeckelement sind grundsätzlich flüssigkeitsdicht miteinander verbunden.

In einer Ausgestaltung kehrt die Wandung nach Wegfall des manuellen Drucks durch eine elastische Rückstellkraft zumindest im Wesentlichen in ihre ursprüngliche Form zurück. Eine elastische Rückstellkraft meint das Bestreben eines Bauteils oder eines Teils davon, sich in die ursprüngliche Position bzw. Form zurück zu bewegen. Die Rückstellkraft wird insbesondere durch die Elastizität der Wandung hervorgerufen. Die Wandung ist bestrebt, in ihre ursprüngliche Gestalt zurückzukehren. Auf diese Weise wird ein Unterdruck im Dosierungshohlraum erzeugt. Bei Wegfall der äußeren Kraft kann die Wandung in ihre ursprüngliche Gestalt zurückkehren. Insbesondere strömt dabei Flüssigkeit durch den Einlass in den Dosierungshohlraum, bevorzugt angesaugt durch den entstehenden Unterdruck. Diese Ausgestaltung ermöglicht ein einfaches Auffüllen des Dosierungshohlraums und eine reproduzierbare Dosierung.

In einer Ausgestaltung umfasst die Dosierungseinrichtung ein erstes Rückschlagventil, das ein Strömen der Flüssigkeit vom Dosierungshohlraum zum Einlass verhindert, und/oder ein zweites Rückschlagventil, das ein Strömen der Flüssigkeit vom Auslass zum Dosierungshohlraum verhindert. Ein Rückschlagventil ist ein Bauteil, welches die Strömung einer Flüssigkeit in nur einer Richtung zulässt. Das erste Rückschlagventil ist typischerweise so ausgebildet und angeordnet, dass es die Strömung der Flüssigkeit nur durch den Einlass in den Dosierungshohlraum zulässt. Das zweite Rückschlagventil ist typischerweise so ausgebildet und angeordnet, dass es die Strömung der Flüssigkeit nur aus dem Dosierungshohlraum zum Auslass zulässt. Auf diese Weise wird auf einfache Weise ein Auffüllen des Dosierungshohlraums ermöglicht.

Das erste Rückschlagventil kann als Kugelventil, Klappenventil und/oder als Lippenventil ausgebildet sein. Das zweite Rückschlagventil kann als Kugelventil, Klappenventil und/oder als Lippenventil ausgebildet sein. Ein beweglicher Ventilkörper des Ventils wie z. B. eine Kugel eines Kugelventils oder eine Klappe eines Klappenventils kann eine größte Erstreckung oder einen Durchmesser von höchstens 1 mm aufweisen. Derartige Ventile haben sich in dem vorliegenden Größenbereich als dauerhaft und verlässlich erwiesen, reagieren nicht mit Wirkstoffen und sind kostengünstig herstellbar. Insbesondere können die Rückschlagventile in dafür vorgesehene Hohlräume des Gehäuses der Vorrichtung eingeschoben werden. Insbesondere können Rückschlagventile dort verrastet oder eingeschraubt werden. Dazu weisen Rückschlagventile an ihrer Außenseite insbesondere geeignete Formelemente zum Schrauben oder Verrasten auf.

In einer Ausgestaltung umfasst die Vorrichtung ferner einen Vorratshohlraum zur Aufnahme eines Vorrats der zu dosierenden Flüssigkeit. Der Vorratshohlraum steht in Fluidverbindung mit dem Einlass. Ein Vorratshohlraum ist ein Hohlraum, der als Vorrat dient. Der Vorratshohlraum kann über ein Leitungselement mit dem Dosierungshohlraum bzw. mit dem Einlass verbunden sein.

In einer Ausführungsform ist der Vorratshohlraum fest mit dem Einlass und/oder mit dem Dosierungshohlraum verbunden. Auf diese Weise kann eine besonders kompakte Vorrichtung bereitgestellt werden, die sowohl einen Vorrat als auch die Dosierungseinrichtung umfasst. Insbesondere bei der Verwendung als Implantat ist dies von Vorteil. In einer Ausführungsform umfasst die Wandung des Vorratshohlraums eine im Wesentlichen starre Bodenplatte und ein mit dieser mechanisch verbundenes elastisches Abdeckelement. In einer Ausführungsform ist zumindest ein Teil einer den Vorratshohlraum begrenzenden Wandung einstückig mit zumindest ein Teil einer den Dosierungshohlraum begrenzenden Wandung hergestellt. Beispielsweise ist eine gemeinsame Bodenplatte und/oder ein gemeinsames Abdeckelement für den Vorratshohlraum und den Dosierungshohlraum vorgesehen. Auf diese Weise kann die Vorrichtung mit geringem technischem Aufwand hergestellt werden. Beispielsweise kann das Abdeckelement auf ein Gehäuse oder eine Bodenplatte aufgeklebt oder aufgeschweißt sein. In einer Ausführungsform sind der Dosierungshohlraum und der Vorratshohlraum einander benachbart angeordnet. Typischerweise beträgt ein Abstand zwischen dem Dosierungshohlraum und dem Vorratshohlraum und/oder die Länge eines dazwischen angeordneten Leitungselements höchstens 15 mm, insbesondere höchstens 10 mm, vorteilhaft höchstens 5 mm und bevorzugt höchstens 3 mm.

Insbesondere ist ein Volumen des Vorratshohlraums wenigstens um den Faktor 2, bevorzugt Faktor 5, größer ist als ein Volumen des Dosierungshohlraums. Auf diese Weise kann ein mehrfaches Dosieren ermöglicht werden, ohne dass der Vorratshohlraum zwischenzeitlich aufgefüllt werden muss. Insbesondere ist ein Volumen des Vorratshohlraums höchstens um den Faktor 50, typischerweise um den Faktor 25 und in einem Beispiel um den Faktor 15 oder 10 größer als das Volumen des Dosierungshohlraums. Ein im Vergleich zu großer Vorratshohlraum geht mit einer übermäßig großen Vorrichtung einher, die praktische Probleme mit sich bringt. Die unterschiedlichen Größenordnungen des Faktors resultieren aus den sehr unterschiedlichen zu dosierenden Volumina unterschiedlicher Wirkstoffe. Bevorzugt hat der Dosierungshohlraum ein nutzbares Volumen zwischen 20 µl und 200 µl. In Abhängigkeit des zu dosierenden Wirkstoffs, der gewünschten Dosiermenge und der Wirkstoffkonzentration in der Flüssigkeit sind jedoch auch kleinere oder größere Volumina möglich. Insbesondere hat der Vorratshohlraum ein Volumen größer als 50 µl, insbesondere als 100 µl und bevorzugt als 200 µl und/oder kleiner als 20 ml, insbesondere als 10 ml und bevorzugt als 5 ml. Insbesondere beziehen sich die Angaben zum Volumen des Vorratshohlraums auf ein nutzbares Volumen des Vorratshohlraums, welches dem maximalen Flüssigkeitsvolumen des Vorratshohlraums abzüglich einem etwaig im Vorratshohlraum verbleibenden Flüssigkeitsvolumen entspricht. Der Vorratshohlraum hat typischerweise eine maximale Erstreckung, z. B. einen maximalen Durchmesser von 30 mm.

Insbesondere ist der Vorratshohlraum scheibenförmig und/oder hat in Draufsicht eine kreisrunde Form. Insbesondere hat der Vorratshohlraum eine plane Unterseite und/oder eine konvex gewölbte Oberseite. Die Form des Vorratshohlraums kann als plan-konvex beschrieben werden.

In einer Ausgestaltung ist der Vorratshohlraum durch eine zumindest bereichsweise elastisch verformbare Wandung gebildet, sodass sich der Vorratshohlraum bei abnehmendem Vorrat der Flüssigkeit verkleinert. Auf diese Weise wird verhindert, dass sich Luft oder ungewünschte Flüssigkeit im Vorratshohlraum sammelt, wenn der Vorrat der zu dosierenden Flüssigkeit abnimmt. Zudem kann der Vorratshohlraum auf diese Weise ähnlich dem Dosierungshohlraum und ggf. zusammen mit diesem hergestellt werden.

Insbesondere besteht die Wandung zumindest einer Seite des Vorratshohlraums zumindest im Wesentlichen aus einem elastisch verformbaren Material. In einer Ausführungsform besteht die Wandung des gesamten Vorratshohlraums teilweise oder vollständig aus einem elastisch verformbaren Material. So kann bei Änderung des Füllstands des Vorratshohlraum auf einfache Weise eine Volumenänderung erfolgen.

In einer Ausgestaltung ist die elastische Rückstellkraft des Dosierungshohlraums größer als eine elastische Rückstellkraft des Vorratshohlraums. Insbesondere ist die elastische Rückstellkraft des elastisch verformbaren Bereichs der Wandung des Dosierungshohlraums größer als die elastische Rückstellkraft des elastisch verformbaren Bereichs der Wandung des Vorratshohlraums. Auf diese Weise kann sichergestellt werden, dass sich das zumindest teilweise verringerte Volumen des Vorratshohlraums nach der Dosierung bzw. Abgabe der Flüssigkeit wieder vergrößert und dabei Flüssigkeit aus dem Dosierungshohlraum angesaugt wird. Es kann auf diese Weise nach erfolgter Dosierung auf besonders einfache Weise Flüssigkeit aus dem Vorratshohlraum in den Dosierungshohlraum gepumpt werden, um anschließend erneut dosieren zu können. Dies geschieht, ohne dass ein Druck oder eine äußere Kraft aufgebracht werden muss. Dies erfolgt auch dann, wenn dadurch eine Verformung des Vorratshohlraums bzw. dessen Wandung notwendig ist.

Zumindest ein Teil der größeren Rückstellkraft des Dosierungshohlraums im Vergleich zum Vorratshohlraum kann durch unterschiedliche Materialien erreicht werden. Die Wandung und/oder das Material der Wandung des Dosierungshohlraums kann im Vergleich zur Wandung bzw. zum Material des Vorratshohlraums eine größere Elastizität, etwa einen kleineren Elastizitätsmodul, aufweisen. Das Material der Wandung des Dosierungshohlraums kann eine größere Härte, beispielsweise eine größere Shore-A-Härte, aufweisen als das Material der Wandung des Vorratshohlraums. Bevorzugt weist das Material des Dosierungshohlraums eine Shore-A-Härte größer als 60 auf. Bevorzugt weist das Material des Vorratshohlraums eine Shore-A-Härte kleiner als 60 auf. Im Falle unterschiedlicher Materialien können die Wanddicken gleich oder unterschiedlich sein. Die genannten Unterschiede können sich auf einen jeweils elastischen Teil der Wandung des jeweiligen Hohlraums oder auf die gesamte Wandung beziehen.

Zumindest ein Teil der größeren Rückstellkraft des Dosierungshohlraums im Vergleich zum Vorratshohlraum kann durch unterschiedliche Wanddicken erreicht werden. Die Wanddicke des Dosierungshohlraums kann größer sein als die Wanddicke des Vorratshohlraums. Im Falle unterschiedlicher Wanddicken können die Materialien gleich oder unterschiedlich sein.

In einer Ausgestaltung ist zumindest ein Bereich der Wandung des Vorratshohlraums so ausgebildet, dass eine Kanüle (Injektionskanüle) durch zumindest den Bereich der Wandung des Vorratshohlraums durchgestochen werden kann, um mit der Kanüle Flüssigkeit in den Vorratshohlraum einzubringen.

Mit anderen Worten ist zumindest ein Bereich der Wandung als Durchstichbereich ausgestaltet. Insbesondere ist die Wandung zumindest einer Seite des Vorratshohlraums, insbesondere des gesamten Vorratshohlraums, teilweise oder vollständig aus einem mit einer Kanüle durchstechbaren Material hergestellt. Bevorzugt ist ein elastischer Bereich der Wandung, beispielsweise ein elastisches Abdeckelement, als Durchstichbereich ausgestaltet. Auf diese Weise kann auf einfache Weise und ohne aufwändiges Öffnen des Hohlraums der Vorrat aufgefüllt werden. Dies ist besonders vorteilhaft bei der Verwendung als Implantat, wobei die Kanüle durch die das Implantat bedeckende Haut- und ggf. Gewebeschicht hindurchgeführt wird. Beispielsweise kann medizinisches Personal oder der Patient selbst mittels einer herkömmlichen Injektionskanüle oder mit einem geeigneten Pen den Vorrat der Flüssigkeit auffüllen. Die Vorrichtung kann so dauerhaft verwendet werden, ohne dass eine Explantation oder einer Entfernung der Vorrichtung von der Hautoberfläche notwendig ist. Beim Zurückziehen der Injektionskanüle kann sich die erzeugte Perforation in der Wandung insbesondere infolge der Rückstellkraft des elastisch verformbaren Materials verschließen.

In einer Ausgestaltung weist die Vorrichtung ein durchstechfestes Element auf, sodass ein ungewünschtes Durchstechen eines rückwärtigen Bereichs der Wandung verhindert wird. Der rückwärtige Bereich ist der dem zu durchstechenden Bereich der Wandung gegenüberliegende Bereich oder der hintere Bereich des Vorratshohlraums. Insbesondere ist das durchstechfeste Element am rückwärtigen Bereich der Wandung angeordnet. Insbesondere ist die Wandung des Vorratshohlraums gemeint. Beispielsweise ist das durchstechfeste Element an einer Bodenplatte der Vorrichtung angeordnet. Das durchstechfeste Element ist für manuell bzw. mit einem Pen bediente Injektionskanülen durchstechfest. Es stoppt die Bewegung der Injektionskanüle. Dadurch werden ein Durchstechen des Bodens des Vorratshohlraums, eine Verletzung des unter dem Boden liegenden Gewebes sowie eine Beschädigung der Vorrichtung sicher verhindert.

In einer Ausgestaltung ist das durchstechfeste Element eine durchstechfeste Platte. Dies ermöglicht mit besonders geringem Aufwand einen Schutz vor ungewünschtem Durchstechen. In einer Ausgestaltung ist das durchstechfeste Element aus einem biokompatiblen Material hergestellt. Auf diese Weise werden Wechselwirkungen mit dem Körper des Patienten sowie mit der Flüssigkeit und darin enthaltenen Wirkstoffen verhindert. Das durchstechfeste Element besteht bevorzugt aus Metall. Insbesondere umfasst das durchstechfeste Element Titan, Titanlegierungen, Tantal, Tantallegierungen, Silber, Silberlegierungen und/oder Edelstahl oder besteht aus einem oder mehreren der genannten Materialien.

In einer Ausgestaltung ist das durchstechfeste Element zumindest teilweise im Vorratshohlraum angeordnet. Zumindest teilweise im Vorratshohlraum meint, dass zumindest ein Teil einer Oberfläche des durchstechfesten Elements mit der im Vorratshohlraum befindlichen Flüssigkeit in Kontakt ist. Dies ermöglicht eine besonders einfache Herstellung. In einer Ausführungsform ist zumindest ein Teil der Oberfläche des durchstechfesten Elements mit Silber beschichtet. Das Silber kann durch Freisetzung von Silber-Ionen antiseptisch wirken. So kann die im Vorratshohlraum befindliche Flüssigkeit gegenüber einer mikrobiellen Kontamination geschützt werden.

In einer Ausführungsform umfasst die Vorrichtung einen oder mehrere mit dem Auslass verbundene oder verbindbare Leitungselemente aus einem elastisch verformbaren Material, insbesondere Kunststoff. Ein oder mehrere Leitungselemente können als Schlauch vorgesehen sein. Jedes Leitungselement weist insbesondere einen äußeren Durchmesser ≤ 3 mm auf. Wenigstens ein Leitungselement ist insbesondere implantierbar, um im Körper möglichst nah an den vorgesehenen Freisetzungsort positioniert zu werden.

In einer Ausführungsform umfassen ein oder mehrere der Leitungselemente voneinander beabstandete Perforationen, wobei die Perforationen insbesondere entlang der Längsachse angeordnet sind. Jede Perforation wird bei Druckbeaufschlagung durch eine im Leitungselement befindliche Flüssigkeit geöffnet, sodass die unter Druck stehende Flüssigkeit austritt. Jede Perforation verschließt sich automatisch nach Beendigung der Druckbeaufschlagung, insbesondere durch die Rückstellkraft des elastischen Materials. Die Leitungselemente sind bevorzugt gemäß der Lehre der Offenlegungsschrift EP3795196A1 und/oder EP3795196A1 ausgestaltet, insbesondere perforiert. In einer weiteren Ausführungsform ist am Ende eines oder mehrerer Leitungselemente ein Ventil angeordnet, das bei Druckbeaufschlagung durch die Flüssigkeit reversibel geöffnet wird und das sich nach Beendigung der Druckbeaufschlagung wieder schließt. In beiden genannten Ausführungsformen sind keine dauerhaften Öffnungen an den Leitungselementen vorhanden. Auf diese Weise wird ein Verstopfen, etwa durch Einwachsen von Bindegewebe oder durch Eindringen von Blut, verhindert. Die Leitungselemente können so über einen Zeitraum von Wochen bis zu mehreren Monaten genutzt werden.

In einer Ausgestaltung weist die Vorrichtung eine maximale Höhe kleiner als 13 mm auf, insbesondere kleiner als 10 mm. Die Höhe bezieht sich auf die Gesamthöhe eines Hauptkörpers der Vorrichtung, der insbesondere die Dosierungseinrichtung, den Einlass, den Auslass und ggf. den Vorratshohlraum umfasst. Etwaige Leitungselemente wie an den Auslass angeschlossene Schläuche werden nicht berücksichtigt. Analog dazu beziehen sich die Breite und die Länge auf jeweilige Gesamtmaße des Hauptkörpers. Die maximale Höhe ist die Höhe an der höchsten Position. Dies gilt analog für die Breite und Länge. Die höchste Position bezieht sich auf einen Zustand der Vorrichtung, in dem der Vorratshohlraum und/oder der Dosierungshohlraum oder bestimmungsgemäß vollständig gefüllt ist.

Mit anderen Worten ist die Vorrichtung flach. Auf diese Weise kann die Vorrichtung besonders einfach implantiert werden. Bevorzugt ist die maximale Höhe kleiner als 7 mm. Die maximale Höhe ist typischerweise größer als 4 mm. In einer Ausführungsform weist die Vorrichtung eine im Wesentlichen ebene Unterseite auf. Diese kann durch eine im Wesentlichen ebene Bodenplatte bereitgestellt werden. Die Bodenplatte kann im Wesentlichen steif sein. Auf diese Weise kann die Vorrichtung besonders einfach implantiert werden. Insbesondere ist die Vorrichtung scheibenförmig ausgebildet.

In einer Ausgestaltung ist eine Breite der Vorrichtung wenigstens um den Faktor 3 größer als eine Höhe der Vorrichtung. Insbesondere ist der Faktor größer als 4 und/oder kleiner als 10, bevorzugt kleiner als 6. Es sind jeweils eine maximale Breite und eine maximale Höhe gemeint. In einer Ausgestaltung ist eine Länge der Vorrichtung um wenigstens den Faktor 5 größer ist als eine Höhe der Vorrichtung. Insbesondere ist der Faktor größer als 7 und/oder kleiner als 15, bevorzugt kleiner als 10. Es sind jeweils eine maximale Länge und eine maximale Höhe gemeint.

In einer Ausgestaltung ist die Wandung des Dosierungshohlraums zumindest bereichsweise konvex geformt. Der konvexe Bereich ist bevorzugt der zum Pumpen manuell zu drückende Bereich der Wandung. Auf diese Weise kann der zu drückende Punkt einfach ertastet werden. Dies ist insbesondere von Vorteil, wenn die Vorrichtung als Implantat verwendet wird, da hier ein Ertasten durch die Haut hindurch erfolgt.

Beim Implantieren wird die Vorrichtung und ggf. die ein oder mehreren Leitungselemente unter der Haut angeordnet. Insbesondere wird die mit der Flüssigkeit gefüllte Vorrichtung implantiert. So wird verhindert, dass Luft in der Vorrichtung ist, die beim ersten Dosieren freigesetzt werden könnte. Die Leitungselemente werden typischerweise so positioniert, dass eine Freisetzung von Wirkstoffen an oder in der Nähe eines Bestimmungsorts erfolgen kann. Z. B. wird die Vorrichtung an einem Handrücken implantiert, um Flüssigkeit gezielt rheumatischen Fingergelenken zuführen zu können.

Ein weiterer Aspekt der Erfindung ist ein Verfahren zur dosierten Abgabe einer Flüssigkeit zwecks Wirkstofffreisetzung im menschlichen oder tierischen Körper unter Verwendung einer erfindungsgemäßen Vorrichtung. Durch manuellen Druck auf die Wandung wird der Dosierungshohlraum verkleinert, sodass ein definiertes Volumen der Flüssigkeit aus dem Dosierungshohlraum zum Auslass gefördert wird. Alle Merkmale, Vorteile und Wirkungen der eingangs genannten Vorrichtung gelten auch für das Verfahren und umgekehrt. In einer Ausgestaltung ist die Vorrichtung in dem menschlichen oder tierischen Körper unter der Haut implantiert. In einer alternativen Ausgestaltung ist die Vorrichtung an der Hautoberfläche befestigt, insbesondere festgeklebt. In einer Ausgestaltung ist das Verfahren ein therapeutisches oder ein nicht therapeutisches Verfahren. Die Flüssigkeit kann als Wirkstoff einen oder mehreren aus der Gruppe umfassend Analgetika, nicht-steroidale Antiphlogistika, Glucocorticoide, Basistherapeutika (Disease modifying anti-rheumatic drugs, DMARD), Biologicals und Immunmodulatoren enthalten, bevorzugt solche, die über eine längere Zeit hydrolytisch stabil sind.

Ein weiterer Aspekt der Erfindung ist ein Verfahren zum Auffüllen der erfindungsgemäßen Vorrichtung. Es wird eine Kanüle durch einen Bereich der Wandung des Vorratshohlraums durchgestochen, um mit der Kanüle Flüssigkeit in den Vorratshohlraum einzubringen. Alle Merkmale, Vorteile und Wirkungen der oben genannten Aspekte gelten auch für das Verfahren und umgekehrt.

Ein weiterer Aspekt der Erfindung ist die Verwendung einer erfindungsgemäßen Vorrichtung zur dosierten Abgabe einer Flüssigkeit zwecks Wirkstofffreisetzung im menschlichen oder tierischen Körper. Insbesondere wird durch manuellen Druck auf die Wandung der Dosierungshohlraum verkleinert, sodass ein definiertes Volumen der Flüssigkeit aus dem Dosierungshohlraum zum Auslass gefördert wird. Insbesondere ist die Vorrichtung in einem menschlichen oder tierischen Körper unter der Haut implantiert oder an der Hautoberfläche befestigt, insbesondere festgeklebt. In einer Ausgestaltung ist die Verwendung eine therapeutische oder eine nicht therapeutische Verwendung. Alle Merkmale, Vorteile und Wirkungen der oben genannten Aspekte gelten auch für die Verwendung und umgekehrt.

Nachfolgend werden Ausführungsbeispiele der Erfindung auch anhand von Figuren näher erläutert. Merkmale der Ausführungsbeispiele können einzeln oder in einer Mehrzahl mit den beanspruchten Gegenständen kombiniert werden, sofern nichts Gegenteiliges angegeben wird. Die beanspruchten Schutzbereiche sind nicht auf die Ausführungsbeispiele beschränkt.

Es zeigen:
- Figur 1:: eine perspektivische Ansicht einer erfindungsgemäßen Vorrichtung,
- Figur 2:: eine Draufsicht auf eine erfindungsgemäße Vorrichtung,
- Figur 3:: eine Seitenansicht einer erfindungsgemäßen Vorrichtung,
- Figur 4:: eine Schnittzeichnung einer erfindungsgemäßen Vorrichtung bei der Durchführung eines Verfahrensschrittes,
- Figur 5:: eine Schnittzeichnung einer erfindungsgemäßen Vorrichtung bei der Durchführung eines weiteren Verfahrensschrittes,
- Figur 6: ein vergrößertes Detail aus Figur 5,
- Figur 7: eine perspektivische Ansicht einer erfindungsgemäßen Vorrichtung,
- Figur 8: eine Schnittzeichnung einer erfindungsgemäßen Vorrichtung beim Auffüllen, sowie
- Figur 9: eine Schnittzeichnung einer erfindungsgemäßen Vorrichtung nach dem Auffüllen.

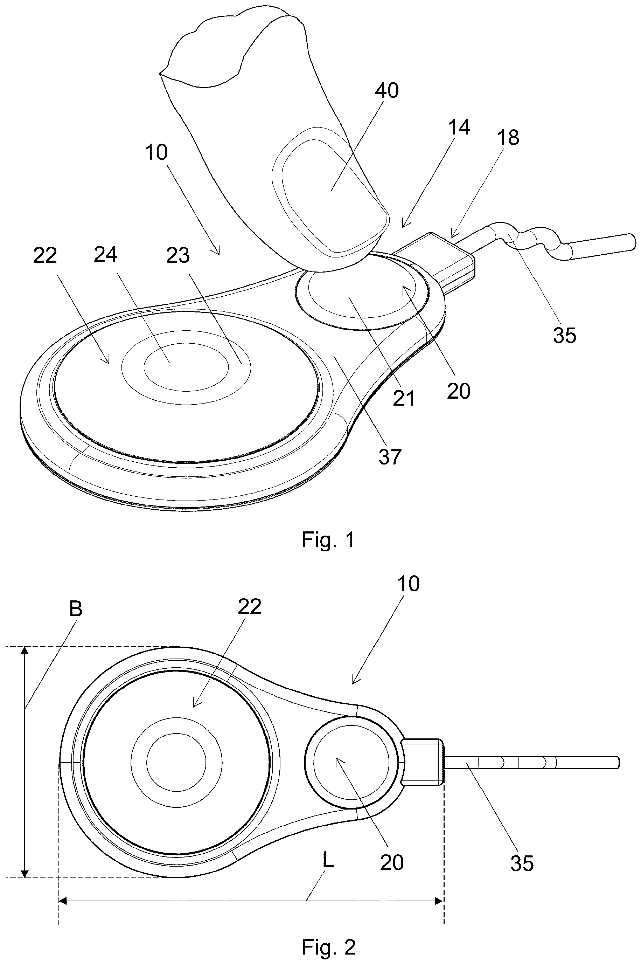

Figur 1 zeigt eine Vorrichtung 10 zur dosierten Abgabe einer Flüssigkeit zwecks Wirkstofffreisetzung im menschlichen oder tierischen Körper. Die Vorrichtung 10 umfasst ein Gehäuse 37, in dem eine Dosierungseinrichtung 14 untergebracht ist. Die Dosierungseinrichtung 14 umfasst einen Dosierungshohlraum 20, der nach oben hin von einer elastischen Wandung 21 begrenzt wird. Durch manuellen Druck mit einem Finger 40 auf die Wandung 21 kann ein definiertes Volumen einer Flüssigkeit aus dem Dosierungshohlraum 20 aus einem Auslass 18 der Vorrichtung ausgegeben werden. Mit dem Auslass 18 ist ein Leitungselement, nämlich ein Schlauch 35 verbunden, durch den das dosierte Volumen zu einer Zielposition geführt werden kann.

Der Dosierungshohlraum 20 umfasst als Einlass eine strömungstechnische Verbindung zu einem Vorratshohlraum 22, der ebenfalls im Gehäuse 37 untergebracht ist. Auch der Vorratshohlraum 22 ist nach oben hin von einer elastischen Wandung 23 begrenzt. Nach erfolgter Dosierung der Flüssigkeit kann somit Flüssigkeit aus dem Vorratshohlraum 22 in den Dosierungshohlraum 20 strömen, um eine erneute Dosierung zu ermöglichen.

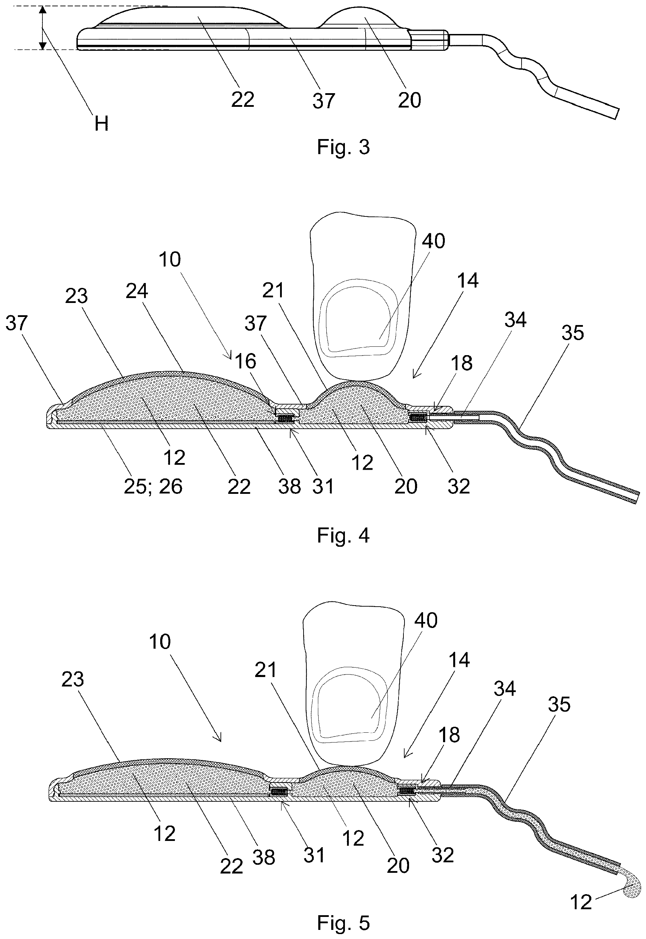

Figur 2 zeigt eine Draufsicht und Figur 3 zeigt eine Seitenansicht. Es ist ersichtlich, dass sowohl die Breite B als auch die Länge L der Vorrichtung 10 deutlich größer sind als die Höhe H der Vorrichtung 10. Die Breite B der Vorrichtung 10 ist um einen Faktor zwischen 5 und 6 größer als die Höhe H der Vorrichtung. Die Länge L der Vorrichtung 10 ist um einen Faktor zwischen 8 und 9 größer als die Höhe H der Vorrichtung. Die Höhe H der Vorrichtung beträgt zwischen 5 mm und 7 mm. Insgesamt ist die Vorrichtung 10 flach, plattenförmig und kompakt ausgestaltet, um eine Implantation unter die Haut eines Patienten zu ermöglichen. Das nutzbare Volumen des Vorratshohlraums 22 ist insbesondere um einen Faktor zwischen 6 und 10 größer als das nutzbare Volumen des Dosierungshohlraums 20, sodass 6 bis 10 Dosierungsvorgänge möglich sind.

Die Figuren 4 und 5 zeigen einen möglichen Aufbau sowie eine Verwendung der Vorrichtung 10 im Längsschnitt. Es sind die Dosierungseinrichtung 14 mit dem Dosierungshohlraum 20 sowie Einlass 16 und Auslass 18 dargestellt. Mit dem Einlass 16 ist der Vorratshohlraum 22 verbunden. Am Auslass 18 befindet sich ein Schlauchanschluss 34 mit einem daran angeschlossenen Schlauch 35. Ein erstes Rückschlagventil 31 ermöglicht einen Flüssigkeitsstrom vom Vorratshohlraum 22 durch den Einlass 16 in den Dosierungshohlraum 20, aber nicht in entgegengesetzter Richtung. Ein zweites Rückschlagventil 32 ermöglicht einen Flüssigkeitsstrom vom Dosierungshohlraum 20 zum Auslass 18, aber nicht in entgegengesetzter Richtung. Auf diese Weise wird eine stets bestimmungsgemäße Strömungsrichtung der Flüssigkeit gewährleistet. Ein Eindringen von dosierter Flüssigkeit oder Körperflüssigkeit in die Vorrichtung wird verhindert.

Das Gehäuse 37 der Vorrichtung umfasst eine Bodenplatte 38 sowie äußere und mittige oberen Gehäuseteile. Das Gehäuse 37 ist aus einem vergleichsweise steifen Kunststoff hergestellt. Die jeweils oberen Bereiche der Wandung 21 des Dosierungshohlraums 20 sowie der Wandung 23 des Vorratshohlraums 22, die am Gehäuse 37 befestigt sind, sind aus einem elastischen Material hergestellt und dementsprechend verformbar.

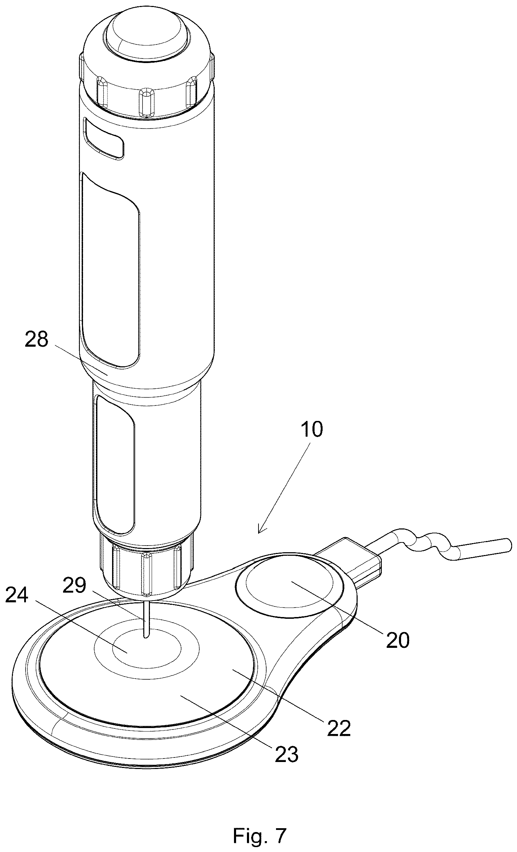

Die elastische Wandung 23 des Vorratshohlraums 22 ist so ausgestaltet, dass sie mit einer Kanüle durchstochen werden kann, um Flüssigkeit 12 in den Vorratshohlraum 22 einzubringen. Dies ist in den Figuren 7 bis 9 dargestellt, auf die weiter unten eingegangen wird. Die Vorrichtung 10 umfasst ferner ein durchstechfestes Element 25, das ein ungewünschtes Durchstechen der Bodenplatte 38 verhindert. Das durchstechfeste Element 25 ist als durchstechfeste Platte 26 aus einem biokompatiblen metallischen Material ausgebildet, die auf der Oberseite der Bodenplatte 38 im Inneren des Vorratshohlraums 22 angeordnet ist. So kann die Vorrichtung im Röntgen dargestellt werden. Die Oberseite der durchstechfesten Platte 26 ist bevorzugt mit Silber beschichtet.

Figur 4 zeigt einen Zustand, in dem sowohl der Dosierungshohlraum 20 als auch der Vorratshohlraum 22 mit der Flüssigkeit 12 gefüllt sind. Die jeweiligen flexiblen Wandungen 21 und 23 sind dabei jeweils konvex nach oben gewölbt. Dies ermöglicht ein besonders einfaches Ertasten des Dosierungshohlraums 20 und damit des Punktes, an dem der manuelle Druck auszuüben ist.

Figur 5 zeigt einen anschließenden Zustand, in dem Flüssigkeit 12 durch manuellen Druck des Fingers 40 auf die Wandung 21 des Dosierungshohlraums 20 durch den Auslass 18, den Schlauchanschluss 34 und den Schlauch 35 abgegeben wird. Das erste Rückschlagventil 31 sperrt, so dass keine Flüssigkeit vom Dosierungshohlraum 20 in den Vorratshohlraum 22 strömen kann. Das zweite Rückschlagventil 32 ist hingegen geöffnet, so dass die Flüssigkeit aus dem Dosierungshohlraum 20 zum Auslass 18 gelangen kann.

Zum einen ist in Fig. 5 im Vergleich zu Fig. 4 die konvexe Wölbung des Dosierungshohlraums 20 und damit dessen Volumen verringert. Dies kann durch weiteres Herunterdrücken des Fingers 40 weiter fortgeführt werden, bis ein Endpunkt erreicht ist, beispielsweise wenn die Wandung 21 zumindest mittig die Bodenplatte 38 berührt. Mit anderen Worten wird der Dosierungshohlraum 20 verkleinert, um das definierte Volumen der Flüssigkeit 12 aus dem Dosierungshohlraum 20 zum Auslass 18 zu fördern. In diesem Fall wurde dann das definierte Volumen der Flüssigkeit 12 dosiert. Zum anderen ist auch die konvexe Wölbung des Vorratshohlraums 22 und damit dessen Volumen verringert. Dies kann beispielsweise deshalb der Fall sein, weil zuvor bereits ein oder mehrere Dosierungsvorgänge durchgeführt worden sind.

Nach erfolgter Dosierung kann der Finger 40 entfernt werden. Durch die elastische Rückstellkraft der Wandung 21 wird im Dosierungshohlraum 20 ein Unterdruck erzeugt, der ein Öffnen des ersten Rückschlagventils 31 bedingt. Auf diese Weise kann Flüssigkeit 12 aus dem Vorratshohlraum 22 in den Dosierungshohlraum 20 nachströmen. Dabei kann die Wandung 23 des Vorratshohlraums 22 verformt werden.

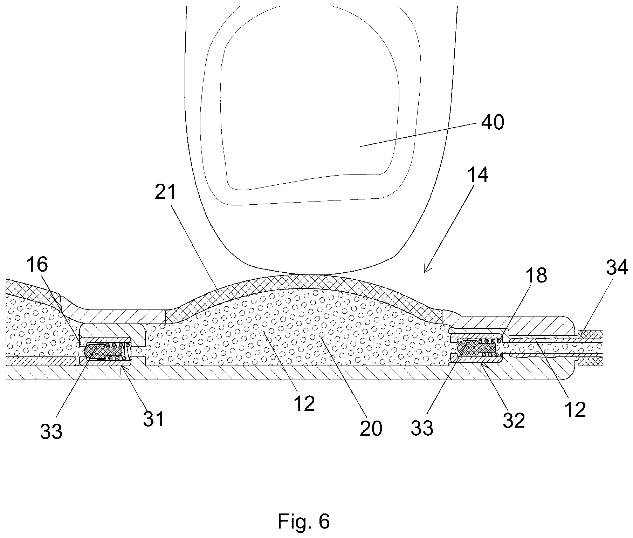

Figur 6 zeigt eine vergrößerte Detaildarstellung aus Figur 5. Es ist ersichtlich, dass sich der Ventilkörper 33 des ersten Rückschlagventils in seiner Schließstellung befindet. Er wird durch die Kraft einer rechts im Ventil angeordneten Druckfeder und gegebenenfalls zusätzlich durch den Druck der Flüssigkeit 12 im Inneren des Dosierungshohlraums 20 nach links gegen ein Ventilgehäuse gedrückt und versperrt somit den Durchfluss durch das Ventil. Der Ventilkörper 33 des zweiten Rückschlagventils 32 befindet sich dagegen in einer Öffnungsstellung. Durch den Druck der Flüssigkeit 12 im Inneren des Dosierungshohlraums 20 wird der Ventilkörper 33 entgegen der Kraft der rechts im Ventil angeordneten Druckfeder nach rechts gedrückt, so das auf der linken Seite eine Lücke zwischen dem Ventilkörper 33 und dem Ventilgehäuse entsteht, durch welche die Flüssigkeit 12 am Ventilkörper 33 vorbei und durch den Auslass 18 ausströmen kann.

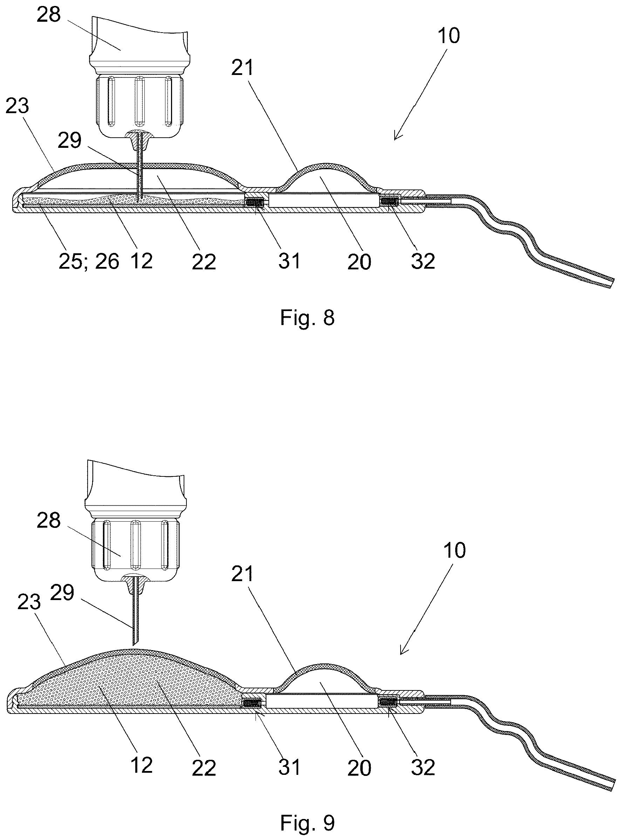

Figur 7 zeigt die erfindungsgemäße Vorrichtung 10 vor dem Befüllen des Vorratshohlraums 22 mit Flüssigkeit. Ein Pen 28 mit einer Kanüle 29 ist unmittelbar oberhalb des durchstechbaren Bereichs 24 der Wandung 23 des Vorratshohlraums 22 angeordnet. Figur 8 zeigt, dass die Kanüle durch die Wandung 23 in den Vorratshohlraum 22 eingestochen worden ist. Die durchstechfeste Platte 26 verhindert ein ungewünschtes Durchstechen des der Wandung 23 gegenüberliegenden Bereichs, hier der Bodenplatte 38. Es ist dargestellt, dass Flüssigkeit 12 aus dem Pen 28 durch die Kanüle 29 in den Vorratshohlraum 22 strömt. Aufgrund der Rückstellkraft der Feder im ersten Rückschlagventil 31 bleibt dieses geschlossen und die Flüssigkeit strömt nicht in den Dosierungshohlraum 20.

Figur 8 zeigt einen anschließenden Schritt, in dem der Vorratshohlraum 22 vollständig gefüllt ist und der Pen 28 mit der Kanüle 29 bereits aus der Wandung 23 herausgezogen worden ist. Die Wandung 23 des Vorratshohlraums 22 ist deutlich nach oben gewölbt. Alternativ zu der hier gezeigten Darstellung kann das Befüllen auch derart erfolgen, dass auch der Dosierungshohlraum 20 mit der Flüssigkeit 12 befüllt wird. Hierzu muss die Flüssigkeit 12 im Vorratshohlraum 22 einen derartigen Druck ausüben, dass das erste Rückschlagventil 31 öffnet. Bevorzugt ist die Federkraft im zweiten Rückschlagventil 32 dann größer bemessen, so dass ein unplanmäßiges Ausströmen der Flüssigkeit durch das zweite Rückschlagventil 32 verhindert wird.

**Bezugszeichenliste**

| | |
|---|---|
| Vorrichtung | 10 |
| Flüssigkeit | 12 |
| Dosierungseinrichtung | 14 |
| Einlass | 16 |
| Auslass | 18 |
| Dosierungshohlraum | 20 |
| Wandung | 21 |
| Vorratshohlraum | 22 |
| Wandung | 23 |
| Bereich | 24 |
| durchstechfestes Element | 25 |
| durchstechfeste Platte | 26 |
| Pen | 28 |
| Kanüle | 29 |
| erstes Rückschlagventil | 31 |
| zweites Rückschlagventil | 32 |
| Ventilkörper | 33 |
| Höhe | H |
| Breite | B |
| Länge | L |
| Schlauchanschluss | 34 |
| Schlauch | 35 |
| Gehäuse | 37 |
| Bodenplatte | 38 |
| Finger | 40 |

## Patentansprüche

1. Vorrichtung (10) zur dosierten Abgabe einer Flüssigkeit (12) zwecks Wirkstofffreisetzung im menschlichen oder tierischen Körper, umfassend eine Dosierungseinrichtung (14) zur Dosierung eines definierten Volumens einer Flüssigkeit (12), einen mit der Dosierungseinrichtung (14) in Fluidverbindung stehenden Einlass (16) zum Zuführen von Flüssigkeit (12) in die Dosierungseinrichtung (14) sowie einen mit der Dosierungseinrichtung (14) in Fluidverbindung stehenden Auslass (18) zur Abgabe des definierten Volumens der Flüssigkeit (12), wobei die Dosierungseinrichtung (14) eine Wandung (21) umfasst, die einen Dosierungshohlraum (20) zur Aufnahme der Flüssigkeit (12) umschließt, **dadurch gekennzeichnet, dass** durch manuellen Druck auf die Wandung (21) der Dosierungshohlraum (20) verkleinert werden kann, sodass das definierte Volumen der Flüssigkeit (12) aus dem Dosierungshohlraum (20) zum Auslass (18) gefördert wird.

2. Vorrichtung (10) nach Anspruch 1, **dadurch gekennzeichnet, dass** die Wandung (21) so ausgebildet ist, dass zumindest ein Bereich der Wandung (21) durch den manuellen Druck elastisch verformt wird und die Wandung (21) nach Wegfall des manuellen Drucks durch eine elastische Rückstellkraft zumindest im Wesentlichen in ihre ursprüngliche Form zurückkehrt.

3. Vorrichtung (10) nach einem der vorhergehenden Ansprüche, **dadurch gekennzeichnet, dass** die Dosierungseinrichtung (14) ein erstes Rückschlagventil (31), das ein Strömen der Flüssigkeit (12) vom Dosierungshohlraum (20) zum Einlass (16) verhindert, und ein zweites Rückschlagventil (32), das ein Strömen der Flüssigkeit (12) vom Auslass (18) zum Dosierungshohlraum (20) verhindert, umfasst.

4. Vorrichtung (10) nach einem der vorhergehenden Ansprüche, **dadurch gekennzeichnet, dass** die Vorrichtung (10) ferner einen Vorratshohlraum (22) zur Aufnahme eines Vorrats der zu dosierenden Flüssigkeit (12) umfasst, wobei der Vorratshohlraum (22) in Fluidverbindung mit dem Einlass (16) steht, wobei ein Volumen des Vorratshohlraums (22) insbesondere wenigstens um den Faktor 2, bevorzugt Faktor 5, größer ist als ein Volumen des Dosierungshohlraums (20).

5. Vorrichtung (10) nach dem vorhergehenden Anspruch, **dadurch gekennzeichnet, dass** der Vorratshohlraum (22) durch eine zumindest bereichsweise elastisch verformbare Wandung (23) gebildet ist, sodass sich der Vorratshohlraum (22) bei abnehmendem Vorrat der Flüssigkeit (12) verkleinert.

6. Vorrichtung (10) nach dem vorhergehenden Anspruch und nach Anspruch 2, **dadurch gekennzeichnet, dass** die elastische Rückstellkraft des Dosierungshohlraums (20) größer ist als eine elastische Rückstellkraft des Vorratshohlraums (22).

7. Vorrichtung (10) nach einem der drei vorhergehenden Ansprüche, **dadurch gekennzeichnet, dass** zumindest ein Bereich (24) der Wandung (23) des Vorratshohlraums (22) so ausgebildet ist, dass eine Kanüle (29) durch die Wandung (23) des Vorratshohlraums (22) durchgestochen werden kann, um mit der Kanüle (29) Flüssigkeit (12) in den Vorratshohlraum (22) einzubringen.

8. Vorrichtung (10) nach dem vorhergehenden Anspruch, **dadurch gekennzeichnet, dass** die Vorrichtung (10) ein durchstechfestes Element (25) aufweist, sodass ein ungewünschtes Durchstechen eines rückwärtigen Bereichs der Wandung (23) des Vorratshohlraums (22) verhindert wird.

9. Vorrichtung (10) nach dem vorhergehenden Anspruch, **dadurch gekennzeichnet, dass** das durchstechfeste Element (25) eine durchstechfeste Platte (26) aus einem biokompatiblen Material ist, die zumindest teilweise im Vorratshohlraum (22) angeordnet ist.

10. Vorrichtung (10) nach einem der vorhergehenden Ansprüche, **dadurch gekennzeichnet, dass** die Vorrichtung (10) eine maximale Höhe (H) kleiner als 13 mm, insbesondere kleiner als 10 mm aufweist.

11. Vorrichtung (10) nach einem der vorhergehenden Ansprüche, **dadurch gekennzeichnet, dass** eine Breite (B) der Vorrichtung (10) wenigstens um den Faktor 3 größer ist als eine Höhe (H) der Vorrichtung (10) und/oder dass eine Länge (L) der Vorrichtung (10) um wenigstens den Faktor 5 größer ist als eine Höhe (H) der Vorrichtung (10).

12. Vorrichtung (10) nach einem der vorhergehenden Ansprüche, **dadurch gekennzeichnet, dass** die Wandung (21) des Dosierungshohlraums (20) zumindest bereichsweise konvex geformt ist.

13. Verfahren zur dosierten Abgabe einer Flüssigkeit (12) zwecks Wirkstofffreisetzung im menschlichen oder tierischen Körper unter Verwendung einer Vorrichtung (10) gemäß einem der Ansprüche 1 bis 12, wobei durch manuellen Druck auf die Wandung (21) der Dosierungshohlraum (20) verkleinert wird, sodass ein definiertes Volumen der Flüssigkeit (12) aus dem Dosierungshohlraum (20) zum Auslass (18) gefördert wird.

14. Verfahren nach dem vorhergehenden Anspruch, **dadurch gekennzeichnet, dass** die Vorrichtung (10) in dem menschlichen oder tierischen Körper unter der Haut implantiert ist.

15. Verwendung einer Vorrichtung (10) gemäß einem der Ansprüche 1 bis 12 zur dosierten Abgabe einer Flüssigkeit (12) zwecks Wirkstofffreisetzung im menschlichen oder tierischen Körper, wobei die Vorrichtung (10) insbesondere in dem menschlichen oder tierischen Körper unter der Haut implantiert ist.
